# EUROPÄISCHE PATENTSCHRIFT

(11) **EP 2 994 074 B1**
(45) Veröffentlichungstag und Bekanntmachung des Hinweises auf die Patenterteilung: **12.09.2018**
(21) Anmeldenummer: 14723057.7
(22) Anmeldetag: 07.05.2014
(51) Int. Cl.: A61F 2/44, A61F 2/30

(54) **WIRBELSÄULENIMPLANTAT**
SPINAL IMPLANT
IMPLANT VERTÉBRAL

(30) Priorität: 07.05.2013 DE 102013208376
(43) Veröffentlichungstag der Anmeldung: 16.03.2016
(73) Patentinhaber: CeramTec GmbH, 73207 Plochingen (DE)
(72) Erfinder: JUSZCZYK, Mateusz, 91235 Velden (DE); KELNBERGER, Alfons, 90552 Röthenbach (DE); MIRUS, Tina, 73732 Esslingen (DE); WECKER, Heinrich, 90542 Eckental (DE); ZIERMANN, Frank, 10967 Berlin (DE)
(74) Vertreter: Fehrenbacher, Eckhard Anton
(86) Internationale Anmeldenummer: PCT/EP2014/059369
(87) Internationale Veröffentlichungsnummer: WO 2014/180917

(56) Entgegenhaltungen:
- EP-A1- 1 790 301
- EP-A1- 2 674 133
- WO-A1-00/49977
- WO-A1-93/01771
- WO-A1-2011/035126
- WO-A2-2004/080356
- WO-A2-2006/119088
- CN-A- 102 144 942
- DE-A1-102010 051 433
- US-A- 5 888 222
- US-A1- 2005 177 238
- US-A1- 2009 254 182
- US-A1- 2010 168 798
- US-A1- 2012 209 385
- US-A1- 2013 090 735

## Beschreibung

Die Offenbarung betrifft Implantate mit Verankerungselementen. Insbesondere betrifft die Offenbarung Wirbelsäulen-Implantate, die als Bandscheibenersatz in Form von Cages zur Fusionierung von Wirbelkörpern verwendet werden können.

Endoprothetische Bauteile zur Fusionierung von Wirbelkörpern sind bekannt. Sie sind in ihrer Geometrie der Anatomie des menschlichen Wirbelkörpers angepasst, befinden sich zwischen zwei Wirbelkörpern und ersetzen die Bandscheibe ganz oder teilweise.

Typischerweise halten sie in einer ersten Phase des Verbleibs im menschlichen Körper allein durch ihre mechanischen Eigenschaften die Wirbelkörper auf Distanz und so in einer anatomisch korrekten Position. In einer zweiten Phase fördern sie die Fusionierung und somit das Verwachsen der beiden sie umgebenden Wirbelkörper.

Bekannte Bauteile zur Fusionierung von Wirbelkörpern basieren auf metallischen Werkstoffen wie z.B. Tantal oder Titan, auf Kunststoffen wie z.B. hochvernetzten PE-Werkstoffen (Polyethylen) oder PEEK (Polyetheretherketon) oder auf Siliziumnitrid. Metallische Werkstoffe haben beispielsweise folgende Nachteile:
- metallischer Abrieb und daraus resultierende negative Auswirkungen auf den menschlichen Organismus, z.B. Fremdkörperreaktionen wie entzündliche oder immunologische Reaktionen, Gewebetoxizität
- Artefakte bzw. fehlende Transluzenz in der Bildgebung bei der medizinischen Diagnostik
- Alterungseffekte und Langzeitverhalten (Ermüdung, Korrosion, Freisetzung von metallischen Ionen, die toxisch wirken können)

Bauteile basierend auf Kunststoffen wie z.B. hochvernetzte PE-Werkstoffe oder PEEK können folgende Nachteile aufweisen:
- Unzureichende mechanische Eigenschaften wie z.B. das Abbrechen von Zacken oder sonstigen Bestandteilen des Bauteils beispielsweise beim Einbau. Dies kann negative Auswirkungen auf den menschlichen Organismus haben.
- Mangelnde Darstellbarkeit bei den gängigen Bildgebungsverfahren (MRI, Röntgen). Dadurch bedingt Einsatz von metallischen Markern.
- Alterungseffekte und Langzeitverhalten, insbesondere Ermüdung des Materials.

Bekannt sind auch keramische Bauteile, beispielsweise basierend auf Siliziumnitrid.

Diese Werkstoffklasse wurde jedoch mit Blick auf exzellente Hochtemperatureigenschaften entwickelt - beispielsweise zur mechanischen Bearbeitung von metallischen Bauteilen für die Automobilindustrie - und rangiert bei den für die Anwendung als medizinisches Implantat geforderten Eigenschaften wie Festigkeit, Härte und Langzeitstabilität im Vergleich zu anderen keramischen Hochleistungswerkstoffen basierend auf oxidischen Systemen eher im Mittelfeld.

Außerdem handelt es sich um einen aus mehreren Komponenten zusammengesetzten Werkstoff mit nadelförmigen Siliziumnitridpartikeln, eingebettet in eine Glasmatrix. Entsprechend aufwändig ist die Sinterung des Werkstoffs. Ebenfalls dadurch bedingt ist die mechanische Bearbeitung wie Schleifen oder Polieren extrem anspruchsvoll und schwierig.

Darüber hinaus weisen aus Si₃N₄ gefertigte Bauteile eine graue bis schwarze Färbung auf, was aus rein optischen und ästhetischen Gründen im medizinischen Bereich auf eine geringe Akzeptanz stößt.

Alle diese Nachteile erhöhen die Kosten bei der Herstellung der Bauteile, was einen weiteren Nachteil darstellt.

Ein grundlegendes Problem, das bei Implantations-Operationen zunehmend in den Fokus rückt, ist das Infektionsrisiko während der Operation. Dieses Risiko kann mit keramischen Bauteilen reduziert werden, deren Oberflächeneigenschaften beispielsweise hemmend auf die Besiedelung mit Bakterien wirken können. Es ist daher wünschenswert verbesserte keramische Implantate, insbesondere für den Einsatz im Wirbelsäulen-Bereich zur Verfügung zu haben.

Bekannte keramische Cages sind in der Regel ringförmig ausgeführt bzw. der Form der menschlichen Wirbelkörper angepasst, wobei der Ring aus einer monolithischen, also dichten, festen und sehr steifen Keramik besteht. Im Zentrum weisen diese Cages einen Hohlraum auf, der entweder mit bekannten Knochenersatzmaterialien (autolog, allogen oder systhetisch) aufgefüllt wird oder eine künstliche poröse osseoinduktive oder osseokonduktive Struktur aufweist. Die osseokonduktive oder osseoinduktive Struktur ist in der Regel wesentlich weniger steif als der äußere Ring. In diesem Bereich sollen Knochenzellen neues Knochenmaterial aufbauen, wobei die daran beteiligten Zellen einen entsprechenden mechanischen Stimulus benötigen.

Aufgrund der relativ komplexen Biomechanik der Wirbelsäule kann es zu sehr unterschiedlichen Belastungszuständen kommen, die sich in Form mikromechanischer Bewegungen oder bedingt durch Beugung oder Streckung der Wirbelsäule in makromechanischen Bewegungen des zu fusionierenden Abschnitts äußern können.

Beide Formen der Bewegung sollten während der Fusion vermieden werden, da sie natürlich auch Auswirkung auf die Position des Implantats und den Heilungsprozess haben können.

Prinzipiell gibt es verschiedene Ansätze zur Vermeidung der Makrobewegungen und entsprechend unterschiedliche Ausgestaltungen der Implantate.

Die Cages können mittels eines separaten Pedikelschrauben- oder mittels eines Platten- und Schraubensystems anterior oder posterior dadurch fixiert werden, dass die angrenzenden Wirbelkörper winkelstabil miteinander verbunden werden, so dass sich beispielsweise bei einer Beugung der Wirbelsäule die Wirbelkörper und das Implantat nicht gegeneinander verschieben und sich unerwünscht separieren. Es werden auch sog. "stand alone" Cages genutzt, die zusätzlich Endplatten mit Aufnahmen oder integrierte Aufnahmen für Schrauben zur direkten Fixierung des Implantats in den Wirbelkörpern aufweisen.

Die zusätzliche Verschraubung des zu fusionierenden Segments bewirkt außerdem, dass die beiden Wirbelkörper und das Implantat mit einer gewissen Presskraft aufeinander liegen, was den Heilungsprozess begünstigen kann und die angestrebte Schmerzfreiheit unterstützt.

Zur Vermeidung von Mikrobewegungen verfügen Cages über zackenförmige Strukturen an der Ober- und/oder Unterseite des Implantats, die sich in den Endplatten der Wirbelkörper verankern und so für eine gewisse Stabilität des Implantats, die sog. Primärstabilität, sorgen.

Diese Verankerungselemente können aber dazu führen, dass die Endplatten der Wirbelkörper beim Einbau geschädigt oder gar zerstört werden, so dass eine Fusion nur sehr eingeschränkt oder gar nicht stattfinden kann bzw. die Cages in die Endplatten der Wirbelkörper einsinken und so das Repositionsergebnis negativ beeinflussen.

Es liegt folgendes Problematik vor: Ist das Verankerungselement zu schwach ausgeprägt, dann schädigt es zwar nicht die Endplatten, sorgt aber nicht für eine ausreichend hohe Primärstabilität. Ist die Verankerung zu stark ausgeprägt, dann schädigt sie beim Einbau die Endplatten mit den oben genannten negativen Folgen.

Die Aufgabe der Erfindung besteht darin, ein Implantat, das insbesondere zur Fusionierung von Wirbelkörpern geeignet ist, bereitzustellen. Das Implantat soll mit Verankerungselementen ausgestattet sein, die eine sichere und schädigungsarme Implantation ermöglichen und gleichzeitig eine sichere Verankerung zwischen den Wirbelkörpern erlauben. Bevorzugt soll das Implantat aus Keramik bestehen.

Ein solches Implantat sollte folgenden Anforderungen genügen:
- Das Implantat sollte während der Implantation die Endplatten der Wirbelkörper nicht nachhaltig schädigen oder zerstören, da dies negative Auswirkungen auf eine erfolgreiche Fusionierung und/oder die mechanische Integrität der Wirbelkörper nach sich ziehen kann.
- Das Implantat sollte durch mechanische Belastungen während der Implantation nicht in einer Weise geschädigt werden, dass es aufgrund von biomechanischen Folgebelastungen zum Bruch des Implantats kommt.
- Das Implantat sollte nach der Implantation eine ausreichend hohe Primärstabilität gewährleisten, d.h. es muss während der Fusion stabil zwischen den Wirbelkörpern verbleiben und darf durch biomechanische Beanspruchung seine Position nicht ändern.
- Das Implantat darf durch biomechanische Belastungen während der Fusion nicht in einer Weise geschädigt werden, dass es zum Bruch des Implantats kommt.
- Das Implantat sollte eine erfolgreiche Stabilisierung des geschädigten Wirbelsäulensegments und eine hohe Fusionsrate der beiden Wirbelkörper gewährleisten.

Die Aufgabe der Erfindung wird durch ein keramisches Wirbelsäulen-Implantat mit den Merkmalen gemäß Anspruch 1 gelöst.

Ein erfindungsgemäßes Implantat weist eine Oberseite, eine Unterseite und eine Mantelfläche auf, wobei die Mantelfläche ggf. in Vorder-, Rück- und Seitenflächen untergliedert sein kann. Ober- und/oder Unterseite weisen Verankerungselemente zur Verbindung mit benachbarten knöchernen Skelett-Elementen auf. Das Implantat ist ein Wirbelsäulen-Implantat. Es handelt sich dabei um einen Cage zur Fusionierung von Wirbelkörpern, wobei die Verankerungselemente zur Verbindung mit den Endplatten von benachbarten Wirbelkörpern dienen.

Gemäß der Erfindung umfasst oder besteht das Implantat aus einer Keramik. Ganz besonders bevorzugt sind Oxidkeramiken, insbesondere aus der Klasse der Aluminiumoxide oder Zirkonoxide oder aus Mischungen von beiden. Besonders vorteilhaft sind extrem schädigungstolerante Werkstoffe wie z.B. eine mit Seltenen Erden, insbesondere Gd und/oder Sa, stabilisierte Dispersoidkeramik. Die Dispersoidkeramik besteht vorzugsweise aus Zirkonoxid, besonders bevorzugt mit Aluminat-Anteilen.

Gemäß einer weiteren bevorzugten Ausführungsform der Erfindung können die Wirbelsäulen-Implantate eine bioaktive Beschichtung aufweisen. Unter einer bioaktiven Beschichtung wird eine Beschichtung verstanden, die mit dem angrenzenden Knochen eine Verbindung eingeht. Solche Beschichtungen können insbesondere aus Hydroxylapatit und/oder Tricalcium-Phosphat bestehen oder diese umfassen. Es eignen sich aber auch Beschichtungen auf Basis von Biogläsern. Andere bioaktive, d.h. beispielsweise osseokonduktiv und/oder osseoinduktiv wirkende Beschichtungen sind ebenfalls möglich. Denkbar sind auch Beschichtungen mit antikmikrobieller Wirkung.

Die Beschichtung der Bauteile dient der Bioaktivierung, die dafür sorgt, dass knochenbildende Zellen gut anhaften, zytokompatible Bedingungen vorfinden und osteogenetisch aktiv werden.

Die Wirbelsäulen-Implantate weisen gemäß der Erfindung eine zentrale Höhlung auf, die sich zumindest durch die Ober- und/oder Unterseite hindurch erstreckt, so dass eine Knochenneubildung von den benachbarten Wirbelkörpern durch das Implantat hindurch stattfinden kann. In solche Höhlungen können vorteilhaft osseokonduktive und/oder osseoinduktive Materialien (autolog, allogen oder künstlich) eingebracht werden, die das Knochenwachstum fördern und/oder ein geeignetes Gerüst für das Einwachsen von Knochenzellen bereitstellen und/oder die Vaskularisierung des neu gebildeten Gewebes fördern.

Um eine gute und nachhaltige Fixierung des Wirbelsäulen-Implantats zu gewährleisten, kann in der Mantelfläche des Implantats zumindest eine Öffnung vorgesehen sein, durch die das Implantat mit zumindest einem benachbarten Wirbelkörper verschraubt werden kann. Die Öffnung sollte so ausgestaltet sein, dass sie keramikgerecht ist, d.h. zum Beispiel scharfe Kanten vermeiden, um Punktbelastungen beim Kontakt mit einer Schraube sicher zu verhindern.

Punktbelastungen können bei Keramiken zum Versagen des gesamten Bauteils durch Bruch führen und sollten deshalb grundsätzlich vermieden werden.

Die Schrauben können aus Metallen oder Metalllegierungen bestehen, die in der Implantationstechnik gängig sind. Besonders bevorzugt können die Schrauben aber ebenfalls aus einem Keramik-umfassenden Material bestehen. Insbesondere bevorzugt ist hier ein Zirkonoxid-umfassendes Material, beispielsweise eine ATZ-Keramik (alumina toughened zirconia). Die Schrauben könnten jedoch ebenfalls aus PEEK oder Polymermaterial bestehen. Das hätte den Vorteil, dass die Problematik von Punktbelastungen der Keramik deutlich gemindert wird. Alle diese Materialien haben darüber hinaus den Vorteil, dass sie bei bildgebenden Untersuchungen nicht zu Artefakten führen und die Darstellbarkeit nicht negativ beeinflussen.

Um eine optimale Passung des Wirbelsäulen-Implantats im Zwischenwirbelraum zu erreichen, können Ober- und/oder Unterseite in Längs- und/oder in Querrichtung konvex gewölbt sein, so dass sie das Bandscheibenfach möglichst weitgehend und passgenau ausfüllen.

Gemäß einer weiteren bevorzugten Ausführungsform können die Ober- und Unterseite im Wesentlichen planparallel zueinander angeordnet sein. Um die Lordose oder die Kyphose einer gesunden Wirbelsäule wieder herstellen zu können, kann es zudem von Vorteil sein, wenn Ober- und Unterseite des Wirbelsäulen-Implantats winklig zueinander angeordnet sind.

Um eine sichere Verankerung des Implantats im Zwischenwirbelraum zu gewährleisten, sieht eine bevorzugte Ausführungsform vor, dass zumindest die gesamte Ober- und/oder Unterseite des Implantats durch Verankerungselemente strukturiert ist. Natürlich können bei entsprechender Ausgestaltung des Implantats auch nur Teilbereiche von Ober- und/oder Unterseite mit Verankerungselementen versehen sein.

Die Verankerungselemente können mit ihrer Längserstreckung senkrecht zur Implantationsrichtung angeordnet sein, so dass eine möglichst große Fläche zur Verfügung steht, die das Implantat entgegen der Implantationsrichtung an seiner Stelle hält. Werden die Verankerungselemente zusätzlich noch in parallelen Reihen angeordnet, kann eine Bewegung in dem durch das Einführen des Implantats geschaffenen Kanal wirksam verhindert werden.

Gemäß einer anderen bevorzugten Ausführungsform der Erfindung können die Verankerungselemente auch in Bögen verlaufen, so dass möglichst große Flächen von Ober- und/oder Unterseite des Implantats mit Verankerungselementen versehen werden können. Diese Anordnung der Verankerungselemente hat den Vorteil, dass erste Teilbereiche der Verankerungselemente das Verrutschen des Implantats entgegengesetzt zur Implantationsrichtung und zweite Teilebereiche der Verankerungselemente das Verrutschen senkrecht zur Implantationsrichtung verhindern. Damit kann also ein Verrutschen des Implantats im Bandscheibenfach nach vorne oder hinten und rechts und links wirksam vermieden werden.

Die bogenförmigen Verankerungselemente können konzentrisch angeordnet sein.

Die Verankerungselemente können auch halbkreisförmig angeordnet sein und/oder Abschnitte von halbkreisförmigen Bögen umfassen. Eine Aneinanderreihung von unterschiedlich ausgerichteten Bögen ergibt eine schlangenlinienartige Anordnung, die ebenfalls unter den Begriff "Bögen" subsummiert wird. Solche Anordnungen der Verankerungselemente haben den gleichen Effekt wie die zuvor beschriebene Ausführungsform.

Als besonders vorteilhafte Form hat sich für die Verankerungselemente eine Rippenstruktur herausgestellt, die im Querschnitt haifischflossenartig sind, d.h. Rippen, die eine längere und eine gegenüberliegende, kürzere Flanke aufweisen. Die kürzere Flanke hat einen steileren Winkel, so dass die Flanke als Widerlager gegen ein Verrutschen dienen kann. In Richtung der langen Flanke ist dagegen ein Einschieben in ein Bandscheibenfach problemlos möglich. Das erfindungsgemäße Wirbelsäulen-Implantat besteht aus zumindest zwei zusammensteckbaren Bauteilen, einem ersten und einem zweiten Bauteil. Ein solches Implantat weist eine erste und eine zweite Außenkontur auf. Die erste Außenkontur hat einen größeren Platzbedarf als die zweite Außenkontur und wird gemäß einer besonders bevorzugten Ausführungsform der Erfindung durch die nur teilweise oder unvollständig zusammengesteckten Bauteile gebildet. Die Verankerungselemente des ersten Bauteils sind in Ausnehmungen des zweiten oder eines weiteren Bauteils und die Verankerungselemente des zweiten Bauteils sind in Ausnehmungen des ersten oder eines weiteren Bauteils zum Implantieren derart aufgenommen, dass die erste Außenkontur des Wirbelsäulen-Implantats im Wesentlichen glatt ist.

Die Bauteile können in diesem Zustand beispielsweise durch Abstandshalter oder vorteilhaft auch durch das Implantationsgerät auf Abstand zueinander gehalten werden, so dass die Verankerungselemente nicht über die sonstigen Oberflächen des Implantats, insbesondere die Ober- und/oder die Unterseite hinausragen.

Dies hat den Vorteil, dass sich die Verankerungselemente bei Implantieren nicht im Gewebe verhaken und/oder das Gewebe beschädigen können. Zwar weist das Bauteil mit der ersten Außenkontur eine größere Höhe auf als das Bauteil mit der zweiten Außenkontur; dafür lässt es sich jedoch problem- und verletzungslos in ein Bandscheibenfach einschieben.

Das Implantat mit der zweiten Außenkontur hat einen geringeren Platzbedarf als das Bauteil mit der ersten Außenkontur und entspricht dem Implantat nach der Implantation, sozusagen im "Funktionszustand". Die Außenkontur ist dadurch geprägt, dass die Verankerungselemente über die sonstigen Oberflächen des Implantats, insbesondere die Ober- und/oder die Unterseite hervorstehen und sich mit den Endplatten der angrenzenden Wirbelkörper verrutschsicher verbinden können. Das geringere Volumen ergibt sich gemäß bevorzugter Ausführungsformen dieses Implantats durch Zusammenschieben der Bauteile, insbesondere in vertikaler Richtung. Unter Zusammenschieben oder Verschieben der Bauteile soll im Rahmen dieser Erfindung ein horizontales, vertikales oder transversales Verschieben verstanden werden. Eine Drehung eines der Bauteile soll jedoch von diesem Begriff nicht erfasst sein.

Aus dem Stand der Technik sind expandierbare Cages aus Metall bekannt, die Lösungen vorsehen, bei denen sich mit Schneidkanten bewehrte Spitzen durch Drehung um eine horizontale Achse nach der Implantierung in die Endplatten der benachbarten Wirbelkörper einschneiden.

Die aus dem Stand der Technik bekannte Lösung kann mit einem keramischen Bauteil jedoch nicht realisiert werden, weil vergleichsweise filigrane Teile wie die Verankerungselemente aus Spitzen mit Schneiden beim Drehen großen, für Keramiken ungeeigneten Belastungen ausgesetzt wären. Es ist damit zu rechnen, dass die Keramikspitzen den Biege- bzw. Zugbelastung nicht oder nur ungenügend standhalten könnten und ein Bauteilversagen die Folge sein könnte.

Im Gegensatz dazu ist die hier vorgeschlagene Lösung mit "ausfahrbaren" Spitzen oder Verankerungselementen eine keramikgerechte Lösung, die auf Biege- und Zugbelastungen des Implantats verzichtet. Die vorgeschlagene Lösung führt im Wesentlichen zu Druckbelastungen, die Keramik-Bauteile gut abfangen können.

Ein weiterer Vorteil der hier beschriebenen Lösung besteht darin, dass die Endplatten der benachbarten Wirbelkörper auf dem Weg in die Endstellung des Implantats nicht mehr als nötig verletzt werden. Die aus dem Stand der Technik bekannte Lösung schneidet durch die Endplatten der Wirbelkörper, um die Spitzen der Verankerungselemente in ihre endgültige Position zu bringen. Die hier beschriebene Lösung schiebt die Verankerungselement im Wesentlichen senkrecht zur Fläche der Endplatte in diese hinein, so dass nur an den Eintrittsstellen Verletzungen auftreten können.

Bei der Lösung nach dem Stand der Technik stellen die Spuren, die durch das Einschneiden der Verankerungselemente entstehen, Schwachpunkte hinsichtlich der Verankerung dar, da in dieser Richtung kein optimales Widerlager für die Verankerungselemente vorhanden ist. Auch diesen Nachteil vermeiden die hier vorgestellten Lösungen.

Gemäß einer besonders bevorzugten Ausführungsform der Erfindung sind das erste und das zweite Bauteil identisch geformt. Dann können die Bauteile so zusammengesteckt werden, dass die Oberseite des ersten Bauteils auf der Oberseite des zweiten Bauteils angeordnet ist. Diese Ausführungsform hat den Vorteil, dass nur ein Bauteil für das gesamte Implantat hergestellt werden muss, was vor allem wirtschaftlich interessant ist.

Die Verankerungselemente dieser Ausführungsform können dornenartige Vorsprünge oder Spitzen sein. Diese Form bietet sich besonders an, weil sie problemlos in Aufnahmen eines anderen Bauteils eingeschoben werden können und gleichzeitig einen guten Halt an den Endplatten der benachbarten Wirbelkörper bieten.

In einer anderen Ausführungsform können die Verankerungselemente dreieckige Vorsprünge sein, die in dreieckige Ausnehmungen des anderen Bauteils durch Einschieben aufgenommen werden können.

Grundsätzlich sind für solche Verankerungselemente alle Formengestaltungen möglich, die sich durch Verschieben, insbesondere in vertikaler Richtung, von einer im Implantat aufgenommenen Position in eine über das Implantat hinausragende Position überführen lassen.

Soll mit dem Wirbelsäulen-Implantat nicht nur eine Bandscheibe, sondern beispielsweise ein ganzer Wirbelkörper ersetzt werden, so ist es auch möglich ein weiteres Bauteil zwischen dem ersten und dem zweiten Bauteil, nach dem Prinzip eines Baukastens anzuordnen. Das weitere Bauteil muss dann die Verankerungselemente bereitstellen, die zum Implantieren in den Ausnahmen des ersten und/oder zweiten Bauteils aufgenommen werden.

Das Implantat weist außerdem vorteilhaft Strukturen auf, mit denen ein Instrument zur Implantation sicher in Eingriff steht.

Die vorbeschriebenen Wirbelsäulen-Implantate können als Bandscheiben-Implantate und insbesondere als Cages für die Fusionierung von benachbarten Wirbelkörper Verwendung finden.

Die Bauteile der Wirbelsäulen-Implantate können entweder als Pressbauteile im grünen Zustand geformt oder in großen Stückzahlen mit Hilfe keramischer Spritzgussverfahren gespritzt werden. Anschließend werden die Bauteile thermisch behandelt, d.h. gesintert, optional gehipt (heißisostatisches Pressen), weißgebrannt, und eventuell die Oberflächen mechanisch nachbehandelt, beispielsweise geschliffen oder poliert.

Die Erfindung wird im Folgenden anhand beigefügter Zeichnungen näher beschrieben. Es zeigen:
- Fig. 1:: Wirbelsäulen-Implantat mit paralleler Rippenstruktur
- Fig. 2:: Wirbelsäulen-Implantat mit konzentrischer Rippenstruktur
- Fig. 3A:: Wirbelsäulen-Implantat mit Schraubenaufnahmen
- Fig. 3B:: Wirbelsäulen-Implantat wie in Fig. 3A mit Schrauben
- Fig. 4A:: Erfindungsgemäßes Wirbelsäulen-Implantat aus zwei Bauteilen mit dornenförmigen Verankerungselementen und einer ersten Außenkontur, die zum Implantieren geeignet ist
- Fig. 4B:: Erfindungsgemäßes Wirbelsäulen-Implantat aus Fig. 4A mit einer zweiten Außenkontur im implantierten Zustand
- Fig. 5:: Erfindungsgemäßes Wirbelsäulen-Implantat aus zwei Bauteilen mit dreieckigen Verankerungselementen

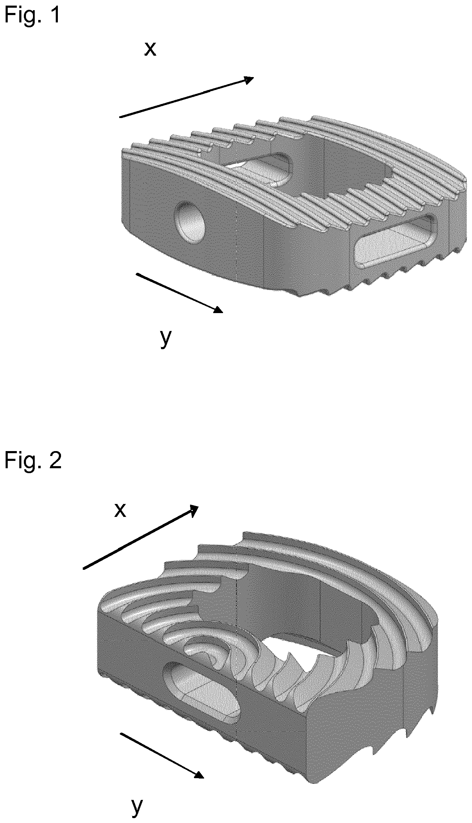

Figur 1 zeigt ein Wirbelsäulen-Implantat in Form eines Cages zur Fusionierung von benachbarten Wirbelkörpern mit Verankerungselementen in Form einer parallelen Rippenstruktur.

Der Cage besteht aus einem Zirkonoxid-verstärkten Al₂O₃-Werkstoff mit hoher Härte und Biegefestigkeit, der sich als biokompatibler Werkstoff in der Medizintechnik etabliert hat.

Die Ober- und Unterseite des Wirbelsäulen-Implantats sind der Anatomie der Wirbelkörper-Endplatten angepasst und weisen in x- und y-Richtung jeweils eine konvexe Fläche auf.

Die Ober- und Unterseiten des Implantats können entweder planparallel zueinander angeordnet sein oder einen Winkel zueinander (Lordose) aufweisen. Diese Ausführung weist einen Lordosewinkel von 7° auf und trägt damit patientenspezifischen anatomischen Anforderungen Rechnung.

Die Verankerungselemente, hier Zacken oder Rippen auf Ober- und Unterseite des Implantats, erstrecken sich über die gesamte Fläche und sind parallel zueinander angeordnet. Sie ermöglichen durch ihre haifischflossenartige Struktur eine, vorzugsweise anteriore, Implantation in x-Richtung und verhindern eine Mikrobewegung in die entgegengesetzte Richtung.

Die Radien der Zacken sind so gestaltet, dass sie einerseits den mechanischen Anforderungen des Werkstoffs und den formgebungstechnischen Möglichkeit des Werkstoffs gerecht werden, andererseits aber auch ein Maximum an Halt und Primärstabilität des Bauteils ermöglichen.

Wird ein Werkstoff mit einer höheren Zähigkeit und Schadenstoleranz, beispielsweise ein Zirkonoxid-basierter Werkstoff, verwendet, lassen sich diese Zackenstrukturen noch ausgeprägter und mit geringeren Radien gestalten, so dass eine noch höhere Primärstabilität erreicht wird.

Die kreisförmige Ausnehmung auf der vorderen Mantelfläche ermöglicht den Eingriff eines Instruments zur sicheren Implantation des Bauteils.

Gleichzeitig kann diese Aussparung auch dazu genutzt werden knochenbildende Materialien in das Innere des Cages einzubringen, beispielsweise in Form eines injizierbaren Zements auf Basis von Hydroxylapatit oder Tricalcium-Phosphat.

Die beiden ovalen Ausnehmungen auf den seitlichen Mantelflächen haben den Vorteil, dass sich darin neu zu bildendes Knochenmaterial sammeln und wachsen kann, was eine zusätzliche Stabilisierung des Bauteils und der fusionierten Wirbelkörper nach sich zieht.

Die Geometrie ist dabei so gewählt, dass eine gewisse kritische Distanz, die die Knochenzellen nicht mehr überbrücken können, nicht überschritten wird (critical size bone defect).

Figur 2 zeigt ein Wirbelsäulen-Implantat mit Verankerungselementen in Form einer konzentrischen Rippenstruktur.

Diese Ausführungsform eines cervicalen Cages ist aus dem gleichen Keramikmaterial hergestellt wie das vorstehende Ausführungsbeispiel, einer Zirkonoxid-verstärkten Al₂O₃-Keramik. Es weist eine andere Zacken- oder Rippenstruktur auf, wobei die Rippen prinzipiell konzentrisch zueinander angeordnet sind und unterschiedliche Radien aufweisen.

Auch diese Struktur ermöglicht die schon beschriebene, schädigungsarme Implantation in der einen Richtung, und verhindert aber nicht nur eine Mikrobewegung in entgegengesetzter Richtung sondern zusätzlich eine Bewegung in y Richtung, also senkrecht zur Implantationsrichtung.

Ein Vorteil ist eine im Vergleich zur vorbeschriebenen Ausführungsform erhöhte Primärstabilität.

Die Zackenstruktur kann im Prinzip auch anders aussehen, wichtig ist nur, dass sie eine zusätzliche Stabilisierung in y Richtung gewährleistet.

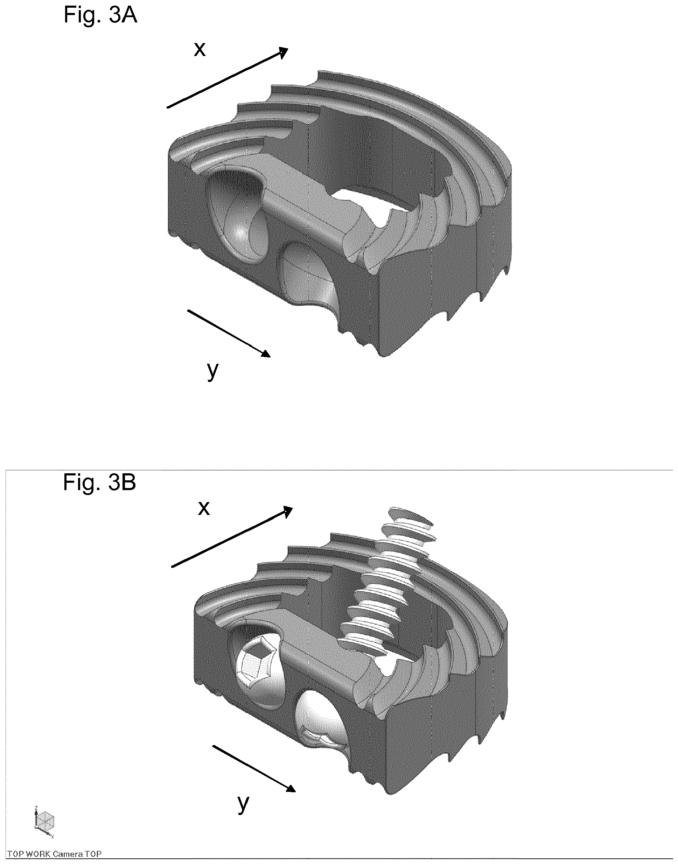

Der gezeigte Cage kann zusätzlich zu der vorstehend beschriebenen Ausführungsform an der vorderen, anterioren Mantelfläche zwei Ausnehmungen aufweisen, siehe Fig. 3A, die Schrauben aufnehmen können, welche zur zusätzlichen Fixierung in die Wirbelkörper geschraubt werden können. Der gleiche Cage mit eingesetzten Schrauben ist in Fig. 3B gezeigt.

Die Schrauben sind vorteilhaft aus Zirkonoxid-basiertem Material, weil sich damit, insbesondere mit Blick auf Zähigkeit und Schadenstoleranz dieses Materials, geeignete und dem Wirbelkörperknochen angepasste Schraubenstrukturen realisieren lassen.

Besondere Aufmerksamkeit ist darauf zu legen, dass beim direkten Kontakt der Schraubenköpfe mit den beiden Ausnehmungen ein Punktkontakt vermieden wird, weil es dadurch zu hohen lokalen Spannungen an diesen Kontaktflächen kommen kann, die in der Folge zum Versagen der Keramik durch Bruch führen können. Darüber hinaus besteht eine Kerbwirkung an scharfen keramischen Bauteil-Kanten, wie sie beispielsweise bei versenkten Schrauben häufig entstehen. Diese Kanten können Ausgangspunkte von Rissen sein, die jedenfalls mittelfristig zum Versagen des Bauteils führen können.

Dieser Punktkontakt kann beispielsweise durch eine besonders keramikgerechte Auslegung der Ausnehmungen vermieden werden, oder durch ein Einlegebauteil aus einem geeigneten Material, beispielsweise Kunststoff, welches die punktuellen Belastungen sicher aufnehmen kann.

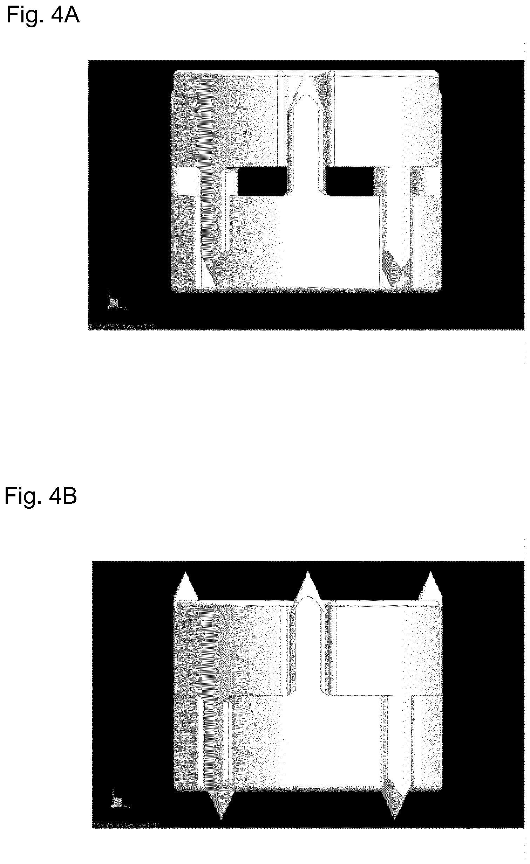

Die Figuren 4A und 4B zeigen ein erfindungsgemäßes Wirbelsäulen-Implantat, das aus zwei Bauteilen mit dornenförmigen Verankerungselementen zusammengesetzt ist. Auch dieses Wirbelsäulen-Implantat kann als Cage zur Fusionierung von Wirbelkörpern verwendet werden.

Es handelt sich also um einen cervicalen Cage, bei dem keine zusätzliche Fixierung zur Vermeidung von Makrobewegungen erforderlich ist.

In einer besonders bevorzugten Ausführungsform umfasst das erfindungsgemäße Implantat zwei identische Bauteile auf. Die beiden Bauteile bilden in Kombination miteinander das erfindungsgemäße Implantat und sind formschlüssig und beweglich miteinander verbunden.

Diese mehrteiligen Cages bestehen ebenfalls bevorzugt aus Keramik, können aber natürlich auch aus anderen gängigen Implantat-Materialien bestehen, beispielsweise PEEK oder Titan bzw. Titanlegierungen.

In einem ersten Zustand der Kombination sind die dornenförmigen Verankerungselemente nicht wirksam, d.h. sie ragen nicht über die jeweiligen Oberflächen (hier: Ober- und Unterseite des Implantats) hinaus. Dies stellt den Zustand während der Implantation dar. Das Implantat weist die definitionsgemäße erste Außenkontur auf.

In einem zweiten Zustand der Kombination liegen die beiden Bauteile flächig aufeinander und die dornenförmigen Verankerungselemente ragen über die jeweiligen Oberflächen hinaus. Dies stellt den Zustand nach der Implantation dar, siehe Fig. 4B. Das Implantat weist die definitionsgemäße zweite Außenkontur auf.

Dieser Mechanismus ermöglicht eine sanfte, zerstörungsfreie Implantation und eine sichere Verankerung durch Verankerungselemente, die sich automatisch nach der Implantation ausfahren.

Wird das Instrument, mit dem das Implantat in das Bandscheibenfach eingeführt wird, entfernt, so verankert sich das Implantat aufgrund der sich unter Last befindenden selbstextrahierenden Zacken selbsttätig und automatisch an der gewünschten Position.

Das Implantat weist außerdem Strukturen auf, mit denen ein Instrument zur Implantation sicher in Eingriff steht und mit denen eine erfindungsgemäße Aufspreizung und Freigabe zur Selbstextraktion möglich ist.

Ein besonderer Vorteil dieser Ausführungsform ist, dass das Implantat bei einer Aufspreizung der beiden Bauteile um einen Weg von x mm dornenförmige Verankerungselemente mit einer Länge von zweimal x mm freigeben kann.

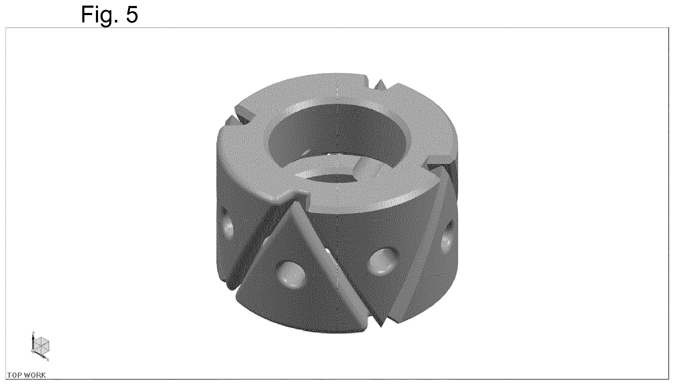

Eine zweite, keramikgerechte Variante, die auf dem gleichen Prinzip wie die Ausführungsform in Fig. 4 beruht, zeigt Fig. 5. Hier sind anstelle der dornenförmigen Verankerungselemente jedoch dreieckige Verankerungselemente vorgesehen.

Das Implantat weist darüber hinaus kreisförmige Löcher oder Ausnehmungen auf, die für die Aufnahme eines Instruments zur Implantation verwendet werden können. Sind diese Löcher entsprechend ausgestaltet, können sie auch für Schrauben zur Fixierung des Bauteils in angrenzenden Wirbelkörpern verwendet werden.

## Patentansprüche

1. Wirbelsäulen-Implantat mit einer Oberseite, einer Unterseite und einer Mantelfläche, wobei das Implantat Verankerungselemente zur Verbindung mit Endplatten von benachbarten Wirbelkörpern aufweist, wobei das Implantat eine zentrale Höhlung aufweist, die sich zumindest durch die Ober- und/oder Unterseite hindurch erstreckt, so dass eine Knochenneubildung von den benachbarten Wirbelkörpern durch das Implantat hindurch stattfinden kann, wobei
das Wirbelsäulen-Implantat zumindest ein erstes und ein zweites Bauteil umfasst, wobei die Bauteile Verankerungselemente aufweisen und zusammensteckbar sind, und das Wirbelsäulen-Implantat eine erste Außenkontur zum Implantieren und eine zweite Außenkontur im implantierten Zustand aufweist, **dadurch gekennzeichnet, dass** das Implantat Keramik umfasst oder aus Keramik besteht und die Verankerungselemente des ersten Bauteils in Ausnehmungen des zweiten Bauteils und/oder die Verankerungselemente des zweiten Bauteils in Ausnehmungen des ersten Bauteils derart aufgenommen sind, dass die Verankerungselemente in der ersten Außenkontur nicht über die Ober- und Unterseite hinausragen und in der zweiten Außenkontur über die Ober und/oder Unterseite hervorstehen, derart, dass sie sich mit den Endplatten der angrenzende Wirbelkörper verbinden und wobei die zweite Außenkontur durch Verschieben der beiden Bauteile gegeneinander aus der erste Außenkontur des Wirbelsäulen-Implantats gebildet wird.

2. Wirbelsäulen-Implantat nach Anspruch 1, **dadurch gekennzeichnet, dass** es aus einer Oxidkeramik und bevorzugt aus einer Oxidkeramik der Klasse der Aluminiumoxide und/oder Zirkonoxide besteht.

3. Wirbelsäulen-Implantat nach einem der vorstehenden Ansprüche, **dadurch gekennzeichnet, dass** es eine bioaktive Beschichtung, insbesondere aus Hydroxylapatit und/oder Tricalcium-Phosphat und/oder eine Beschichtung auf der Basis von Biogläsern aufweist.

4. Wirbelsäulen-Implantat nach einem der vorstehenden Ansprüche, **dadurch gekennzeichnet, dass** die Mantelfläche zumindest eine Öffnung aufweist, durch die das Implantat mit zumindest einem benachbarten Wirbelkörper verschraubt werden kann.

5. Wirbelsäulen-Implantat nach dem vorstehenden Anspruch, **dadurch gekennzeichnet, dass** die Schrauben aus einem Keramik-umfassenden Material, insbesondere einem Zirkonoxid-umfassenden Material bestehen.

6. Wirbelsäulen-Implantat nach einem der vorstehenden Ansprüche, **dadurch gekennzeichnet, dass** die Ober- und/oder die Unterseite in Längs- und/oder in Querrichtung konvex gewölbt ist.

7. Wirbelsäulen-Implantat nach einem der vorstehenden Ansprüche, **dadurch gekennzeichnet, dass** die Ober- und die Unterseite planparallel oder winklig zueinander angeordnet sind.

8. Wirbelsäulen-Implantat nach einem der Ansprüche 1 bis 7, **dadurch gekennzeichnet, dass** ein Volumen der ersten Außenkontur zum Implantieren größer als ein Volumen der zweiten Außenkontur im implantierten Zustand ist.

9. Wirbelsäulen-Implantat nach einem der vorstehenden Ansprüche, **dadurch gekennzeichnet, dass** das erste und das zweite Bauteil identisch geformt sind.

10. Wirbelsäulen-Implantat nach einem der vorstehenden Ansprüche , **dadurch gekennzeichnet, dass** eine Oberseite des ersten Bauteils auf einer Oberseite des zweiten Bauteils angeordnet ist.

## Claims

1. A vertebral-column implant having an upper side, a lower side and a lateral surface, wherein the implant has anchoring elements for connection to end plates of adjacent vertebral bodies, wherein the implant has a central cavity that extends at least through the upper and/or lower side so that bone regeneration of the adjacent vertebral bodies can take place through the implant, wherein the vertebral-column implant comprises at least one first and one second component, wherein the components have anchoring elements and can be plugged together, and the vertebral-column implant has a first outer contour for implantation and a second outer contour in the implanted state, **characterised in that** the implant comprises ceramic material or consists of ceramic material, and the anchoring elements of the first component are received in recesses of the second component and/or the anchoring elements of the second component are received in recesses of the first component in such a way that the anchoring elements in the first outer contour do not project out beyond the upper and lower side and in the second outer contour protrude over the upper and/or lower side in such a way that they are connected to the end plates of the adjoining vertebral bodies, and wherein the second outer contour is formed by displacing the two components against each other out of the first outer contour of the vertebral-column implant.

2. A vertebral-column implant according to claim 1, **characterised in that** it consists of an oxide ceramic material and preferably of an oxide ceramic material of the aluminium-oxide and/or zirconium-oxide class.

3. A vertebral-column implant according to one of the preceding claims, **characterised in that** it has a bioactive coating, in particular of hydroxyapatite and/or tricalcium phosphate, and/or a coating based on bioglasses.

4. A vertebral-column implant according to one of the preceding claims, **characterised in that** the lateral surface has at least one opening through which the implant can be screwed to at least one adjacent vertebral body.

5. A vertebral-column implant according to the preceding claim, **characterised in that** the screws consist of a ceramic-comprising material, in particular a zirconium-oxide-comprising material.

6. A vertebral-column implant according to one of the preceding claims, **characterised in that** the upper and/or the lower side are/is convexly curved in a longitudinal and/or in a transverse direction.

7. A vertebral-column implant according to one of the preceding claims, **characterised in that** the upper and the lower side are arranged so as to be plane-parallel or at an angle to one another.

8. A vertebral-column implant according to one of claims 1 to 7, **characterised in that** a volume of the first outer contour for implantation is greater than a volume of the second outer contour in the implanted state.

9. A vertebral-column implant according to one of the preceding claims, **characterised in that** the first and the second component are shaped identically.

10. A vertebral-column implant according to one of the preceding claims, **characterised in that** an upper side of the first component is arranged on an upper side of the second component.

## Revendications

1. Implant vertébral comprenant une face supérieure, une face inférieure et une surface enveloppe, l'implant présentant des éléments d'ancrage en vue de la liaison avec des plaques d'extrémité de corps vertébraux voisins, l'implant présentant une cavité centrale qui s'étend au moins à travers la face supérieure et/ou la face inférieure, de manière à permettre une néoformation osseuse à travers l'implant, à partir des corps vertébraux voisins,
sachant que
l'implant vertébral comprend au moins un premier et un deuxième composant, les composants présentant des éléments d'ancrage et pouvant être emboîtés l'un dans l'autre, et que l'implant vertébral présente un premier contour extérieur en vue de l'implantation et un deuxième contour extérieur à l'état implanté, **caractérisé en ce que** l'implant comporte de la céramique ou est constitué de céramique, et les éléments d'ancrage du premier composant sont logés dans des évidements du deuxième composant et/ou les éléments d'ancrage du deuxième composant sont logés dans des évidements du premier composant, de manière telle que les éléments d'ancrage dans le premier contour extérieur ne dépassent pas par rapport à la face supérieure et/ou la face inférieure et font saillie par rapport à la face supérieure et/ou la face inférieure, dans le deuxième contour extérieur, de manière à ce qu'ils se raccordent aux plaques d'extrémité des corps vertébraux adjacents, le deuxième contour extérieur étant formé par déplacement des deux composants l'un par rapport à l'autre, à partir du premier contour extérieur de l'implant vertébral.

2. Implant vertébral selon la revendication 1, **caractérisé en ce qu'**il est constitué d'une céramique oxydée et, de préférence, d'une céramique oxydée de la classe des oxydes d'aluminium et/ou des oxydes de zirconium.

3. Implant vertébral selon une des revendications précédentes, **caractérisé en ce qu'**il présente un revêtement bioactif, en particulier en hydroxyapatite et/ou phosphate tricalcique, et/ou un revêtement à base de bioverres.

4. Implant vertébral selon une des revendications précédentes, **caractérisé en ce que** la surface enveloppe présente au moins une ouverture à travers laquelle l'implant peut être vissé à au moins un corps vertébral voisin.

5. Implant vertébral selon la revendication précédente, **caractérisé en ce que** les vis sont constituées d'un matériau comportant de la céramique, en particulier d'un matériau comportant de l'oxyde de zirconium.

6. Implant vertébral selon une des revendications précédentes, **caractérisé en ce que** la face supérieure et/ou la face inférieure est incurvée de manière convexe dans le sens longitudinal et/ou dans le sens transversal.

7. Implant vertébral selon une des revendications précédentes, **caractérisé en ce que** la face supérieure et/ou la face inférieure est disposée à plans parallèles ou en formant un angle l'une par rapport à l'autre.

8. Implant vertébral selon une des revendications 1 à 7, **caractérisé en ce qu'**un volume du premier contour extérieur en vue de l'implantation est plus grand qu'un volume du deuxième contour extérieur à l'état implanté.

9. Implant vertébral selon une des revendications précédentes, **caractérisé en ce que** le premier composant et le deuxième composant ont des formes identiques.

10. Implant vertébral selon une des revendications précédentes, **caractérisé en ce qu'**une face supérieure du premier composant est disposée sur une face supérieure du deuxième composant.
